# FASCICULE DE BREVET EUROPEEN

(11) **EP 3 203 981 B1**
(45) Date de publication et mention de la délivrance du brevet: **13.05.2020**
(21) Numéro de dépôt: 15784090.1
(22) Date de dépôt: 08.10.2015
(51) Int. Cl.: A61K 9/00, A61K 8/67, A61Q 19/08, A61K 8/9789, A61K 31/455, A61K 36/537, A61K 9/06, A61K 9/107, A61K 47/44, A61K 47/10, A61K 47/46

(54) **ACTIVITE DEGLYCATION D'UNE COMBINAISON D'UN EXTRAIT DE SALVIA MILTIORRHIZA ET DE NIACINE ET/OU DE NIACINAMIDE**
DEGLYKIERUNGAKTIVITÄT EINER KOMBINATION VON EINEM SALVIA MILTIORRHIZA-EXTRAKT UND NIACIN UND/ODER NIACINAMIDE
DEGLYCATION ACTIVITY OF A COMBINATION OF AN EXTRACT OF SALVIA MILTIORRHIZA AND NIACIN AND/OR NIACINAMIDE

(30) Priorité: 10.10.2014 FR 1459767
(43) Date de publication de la demande: 16.08.2017
(73) Titulaire: BASF Beauty Care Solutions France S.A.S., 69007 Lyon (FR)
(72) Inventeur: ANDRE, Valérie, 69420 Ampuis (FR); LEOTY-OKOMBI, Sabrina, 38090 Vaulx Milieu (FR); SAGET, Julie, 69008 Lyon (FR)
(74) Mandataire: Cabinet Beau de Loménie
(86) Numéro de dépôt international: PCT/FR2015/052701
(87) Numéro de publication internationale: WO 2016/055737

(56) Documents cités:
- CN-A- 1 539 445
- CN-A- 103 815 202
- US-B2- 7 223 777
- WU ET AL: "One single LC-MS/MS analysis for both phenolic components and tanshinones in Radix Salviae Miltiorrihizae and its medicinal products", TALANTA, ELSEVIER, AMSTERDAM, NL, vol. 73, no. 4, 14 septembre 2007 (2007-09-14), pages 656-661, XP022243160, ISSN: 0039-9140, DOI: 10.1016/J.TALANTA.2007.04.038
- SANG-HUN LEE ET AL: "The protective effect ofin an animal model of early experimentally induced diabetic nephropathy", JOURNAL OF ETHNOPHARMACOLOGY, ELSEVIER IRELAND LTD, IE, vol. 137, no. 3, 2 août 2011 (2011-08-02), pages 1409-1414, XP028324620, ISSN: 0378-8741, DOI: 10.1016/J.JEP.2011.08.007 [extrait le 2011-08-11]
- The Life Extension Foundation Forums: "Glycation inhibition by niacin and L-lysine", , 2001, XP002740423, Extrait de l'Internet: URL:http://forum.lef.org/default.aspx?f=35 &m=16694 [extrait le 2015-06-03]
- LIU X S ET AL: "New alternative therapy for orofacial localized scleroderma", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 110, no. 3, 1 septembre 2010 (2010-09-01), pages e15-e19, XP027366641, ISSN: 1079-2104 [extrait le 2010-07-15]
- Chen et al.,: "Evaluation of a compound with dan-shen root and azone for scar treatment. (PMID 15449627)", PubMed Zhonghua Zheng Xing Wai Ke Za Zhi., mai 2004 (2004-05), XP002740415, Extrait de l'Internet: URL:www.ncbi.nlm.nih.gov/pubmed/15449627 [extrait le 2015-06-02]

## Description

La présente invention concerne un nouvel ingrédient cosmétique et/ou pharmaceutique, notamment dermatologique, et/ou nutraceutique et son utilisation pour augmenter la déglycation du collagène et/ou augmenter l'expression du collagène notamment pour maintenir et/ou augmenter la fermeté et/ou l'élasticité de la peau et/ou des muqueuses et/ou du cuir chevelu et/ou pour prévenir et/ou lutter contre le vieillissement cutané et/ou pour maintenir et/ou augmenter l'éclat du teint de la peau.

La glycation est un mécanisme de glycosylation non enzymatique consistant en la fixation covalente d'un sucre réducteur à une protéine, un lipide ou un acide nucléique. Ce mécanisme conduit à l'apparition de macromolécules plus connues sous le nom de « AGEs » pour « Advanced Glycosylated Endproducts ». Ces molécules sont formées in vivo chez l'humain mais sont aussi apportées par l'alimentation au cours de la préparation alimentaire par chauffage. Ce processus chimique de la glycation aussi connu sous le nom de réaction de Maillard, se déroule en trois étapes : (a) la formation d'une base de Schiff, qui résulte de la fixation d'un sucre réducteur ou d'un aldéhyde sur les résidus aminés des protéines, notamment la lysine au niveau de la partie N-terminale, (b) un réarrangement moléculaire dit d'Amadori, qui résulte d'une isomérisation de la base de Schiff. Le taux de synthèse de ces produits d'Amadori est proportionnel à la concentration en sucre, et (c) l'accumulation des AGEs par réarrangements successifs, transferts d'hydrogène et synthèse d'intermédiaires réactifs. Le taux de synthèse de ces produits n'est pas proportionnel à la concentration en sucre dans le milieu mais est fonction du turn-over protéique.

Les deux premières étapes de ce mécanisme sont réversibles, la dernière est irréversible. Ce mécanisme chimique naturel joue un rôle prépondérant dans le vieillissement cellulaire, et notamment le vieillissement cutané. Au niveau en particulier de la matrice extra-cellulaire (MEC), le mécanisme de glycation diminue la fonctionnalité de protéines telles que le collagène et l'élastine mais il empêche aussi leur renouvellement. La formation de pontages moléculaires entre les fibres de collagène va rendre ces fibres rigides. Ce mécanisme se produit aussi au niveau de la paroi des vaisseaux sanguins, des tendons et muscles squelettiques. De fait, la glycation est impliquée dans diverses maladies liées au diabète et au vieillissement telles que les maladies cardiovasculaires ou l'arthrite.
Au cours du vieillissement cutané, le mécanisme de glycation va entrainer une perte de fermeté, un vieillissement de la peau lié à l'apparition de rides, mais aussi l'apparition d'un teint brouillé et jaune, notamment au niveau du visage. Ces facteurs seront accentués par des facteurs extrinsèques tels qu'une exposition excessive de la peau aux ultraviolets, le tabac, la ménopause, un régime alimentaire riche en sucres et/ou graisses mais aussi certaines pathologies dermatologiques.
Certains ingrédients actifs sont connus pour agir sur les 2 premières étapes de la glycation, réversibles. On qualifie classiquement ces ingrédients actifs de composés antiglycation. Des exemples de ce type de molécules sont l'aminoguanidine, la pyridoxamine ou encore la pioglitazone, et la pentoxyfylline. Le resvératrol et la curcumine (Curcuma longa) ont aussi montré leurs propriétés inhibitrices sur la formation des AGEs.
Toutefois, une fois formés, les AGEs vont s'accumuler de façon irréversible. Il existe par conséquent un réel besoin d'isoler des ingrédients actifs capables d'agir sur cette dernière étape du mécanisme de la glycation. Certains de ces ingrédients actifs sont déjà connus dans l'art antérieur. On parle classiquement de « AGEs breakers », autrement dit d'ingrédients actifs déglycants. Il s'agit d'ingrédients possédant un pouvoir d'attaque nucléophile, capables de rompre les pontages moléculaires établis entre les protéines glyquées et ainsi réverser la réaction de Maillard. Des exemples de ce type de molécules sont la metformine (diméthylbiguanide), la L-bis-4[-(4-chlorobenzamidophenoxyisobutyryl)cystine, l'acide 4-(3,5-dichlorophenylureido)-phenoxyisobutyryl-l-amidocyclohexane-1-carboxylique ou l'acide 1,4-benzene-bis [4-methyleneaminophenoxyisobutyrique]. Un extrait de Rosmarinus officinalis a montré ses propriétés de déglycation. La demanderesse a aussi décrit dans sa demande de brevet sous le numéro FR0756350 les propriétés de réversion de la réaction de glycation de plusieurs ingrédients actifs, molécules et extraits actifs végétaux.
Il existe ainsi un besoin constant dans les domaines de la cosmétique, la pharmacie, notamment la dermatologie, et de la nutraceutique, de mettre à disposition de nouveaux ingrédients alternatifs à ces molécules chimiques et ces ingrédients actifs connus, qui soient des ingrédients actifs naturels capables de déglyquer efficacement les AGEs, notamment les protéines de la MEC.

Le but de la présente invention est de fournir un nouvel ingrédient cosmétique et/ou pharmaceutique, notamment dermatologique, et/ou nutraceutique de type «AGE breaker », capable d'augmenter la déglycation des protéines, notamment des protéines présentes au niveau de la MEC. Il s'agit de fournir un ingrédient cosmétique et/ou pharmaceutique, notamment dermatologique, et/ou nutraceutique pour augmenter la déglycation des protéines de la MEC afin notamment de maintenir et/ou augmenter la fermeté et/ou l'élasticité de la peau et/ou des muqueuses et/ou du cuir chevelu et/ou de prévenir et/ou lutter contre le vieillissement cutané et/ou de maintenir et/ou augmenter l'éclat du teint de la peau.

De façon surprenante, les inventeurs ont découvert que la combinaison d'un extrait de la plante Salvia miltiorrhiza, en particulier de ses parties aériennes, et de niacine et/ou un de ses dérivés, le niacinamide, permet d'augmenter la déglycation des protéines de la MEC, notamment du collagène, de façon plus efficace que respectivement l'extrait de S. miltiorrhiza seul ou que la niacine et/ou le niacinamide, seule. Cette combinaison présente un effet de synergie particulièrement surprenant et inattendu.
Un des avantages de la combinaison selon l'invention est qu'elle augmente par ailleurs l'expression de collagène et ce, de façon tout à fait inattendue, plus efficacement que respectivement l'extrait de la plante S. miltiorrhiza seul ou que la niacine et/ou le niacinamide, seule.
Un autre avantage de cette combinaison encore est qu'elle augmente l'éclat du teint de la peau.
D'autre part, l'ingrédient selon la présente invention est utilisable dans une composition cosmétique et/ou pharmaceutique, notamment dermatologique et/ou nutraceutique. Il est facilement formulable, oralement et/ou topiquement acceptable pour la peau et/ou les muqueuses et/ou le cuir chevelu, en étant par ailleurs stable chimiquement. L'avantage de la présente invention est de fournir un ingrédient cosmétique et/ou pharmaceutique, notamment dermatologique et/ou nutraceutique naturel qui peut être facilement produit à l'échelle industrielle.

La sauge rouge (Salvia miltiorrhiza) est une plante originaire d'Asie, en particulier présente en Chine, en Corée, en Mongolie et au Vietnam. Ses racines sont connues pour leurs effets bénéfiques sur le métabolisme cardiovasculaire, en l'occurrence dans la prévention de disfonctionnements vasculaires et cardiaques, les thromboses cérébrales et l'hypertension. Un traitement de l'insomnie et de l'anxiété par cette plante a aussi été rapporté.
Salvia miltiorrhiza est une plante dont les racines sont connues pour leur activité anti-glycation mais il s'agit en fait d'une préparation inhibitrice d'une alpha-glucosidase (Ma et al., 2011, 65(1):37-42, J. Nat. Med.).
La niacine (acide pyridine-3-carboxylique) (CAS n°59-67-6) est un composé organique de formule brute C₆H₅NO₂. C'est un des nutriments essentiels du corps humain, indispensable à la synthèse des co-enzymes NAD et NADP.
Le niacinamide (CAS n°98-92-0) (C₆H₆NO₂) ou nicotinamide correspond au dérivé amide de la niacine. In vivo, la niacine est convertie en niacinamide. Si les deux molécules ont des fonctions vitaminiques similaires, leurs propriétés pharmacologiques sont différentes. Ainsi, le niacinamide a des propriétés anti-inflammatoires mais ne possède pas l'effet connu vasodilatateur ou encore hypolipidémiant de la niacine. La présence de niacine est aussi connue dans des compositions cosmétiques ou dermatologiques en tant que supplément vitaminique.

La demande de brevet japonaise JP2003012495 décrit une composition pouvant contenir une combinaison d'un acide nicotinique ou un de ses dérivés et d'un extrait de plante, parmi lequel est mentionné un extrait de plante du genre Salvia, sans précision quant à l'espèce particulière utilisée, ledit genre comprenant plus de 250 espèces, pour traiter les dermatites atopiques.
L'article de Wu et al. (Talanta, 73, 2007, pages 656-661), et les demandes CN103815202 et CN1539445 décrivent des compositions contenant un extrait de racines de S. miltiorrhiza avec respectivement l'acide nicotinique et le nicotinamide. Toutefois ces documents ne décrivent pas les utilisations de ces compositions pour augmenter la déglycation des protéines de la MEC, notamment du collagène, pour augmenter l'expression du collagène afin de maintenir et/ou augmenter la fermeté et/ou l'élasticité de la peau et/ou des muqueuses et/ou du cuir chevelu et/ou de prévenir et/ou lutter contre le vieillissement cutané, ni afin de maintenir et/ou augmenter l'éclat du teint de la peau. En effet ces documents ne concernent pas du tout des compositions destinées à une application topique sur la peau et/ou les muqueuses, en particulier destinées à des utilisations cosmétiques.
En outre, il n'est jamais décrit ni suggéré qu'il puisse y avoir une synergie entre les deux composants de la combinaison.
L'article de Sang-Hun Lee et al (Journal of Ethnopharmacology, 137 (2011), pages 1409-1414) décrit l'effet protecteur de l'extrait de racine de de S. miltiorrhiza sur la néphropathie diabétique par administration par voie orale.
Toutefois cet article ne décrit pas de composition destinée à une application par voie topique ni d'association avec la niacine et/ou le niacinamide.
Ainsi, à notre connaissance, un extrait de S. miltiorrhiza et la niacine et/ou le niacinamide n'ont jamais été combinés pour la réalisation d'un ingrédient cosmétique et/ou pharmaceutique, notamment dermatologique, et/ou nutraceutique, ni leur utilisation décrite pour augmenter la déglycation des protéines de la MEC, notamment du collagène, ni pour augmenter l'expression du collagène afin de maintenir et/ou augmenter la fermeté et/ou l'élasticité de la peau et/ou des muqueuses et/ou du cuir chevelu et/ou de prévenir et/ou lutter contre le vieillissement cutané, ni afin de maintenir et/ou augmenter l'éclat du teint de la peau.
En outre la synergie entre ces deux composants n'a jamais été décrite.

Ainsi, la présente invention a pour premier objet une combinaison d'un extrait de Salvia miltiorrhiza, en particulier de ses parties aériennes, et de niacine et/ou de niacinamide.
Au sens de la présente invention, on entend par extrait de S. miltiorrhiza un extrait de tout ou partie de la plante choisi parmi la plante entière, les parties aériennes, la racine, le rhizome, les graines, les fleurs, les pétales, les sépales, les feuilles, le fruit, la tige et/ou l'une quelconque de leur combinaison, plus particulièrement choisi parmi les parties aériennes, les graines, les fleurs, les pétales, les sépales, les feuilles, le fruit, la tige et/ou l'une quelconque de leur combinaison. Préférentiellement, l'extrait selon l'invention est un extrait des parties aériennes de la plante, encore préférentiellement un extrait de feuilles. Au sens de la présente invention, on entend par « parties aériennes » un mélange de feuilles, de tige et fleur de la plante.
L'extrait de S. miltiorrhiza selon l'invention est obtenu par tout procédé classique d'extraction de plantes connu de l'Homme du métier. La plante entière ou la partie de la plante concernée est ainsi séchée et/ou broyée avant extraction. L'extraction peut être réalisée par macération, par décoction à chaud, par broyage à l'aide d'un broyeur à billes, broyage au mortier, aux ultrasons, par broyage à l'aide d'un mixeur ou encore par extraction en conditions subcritiques ou supercritiques (dioxyde de carbone). Préférentiellement, l'extraction est réalisée par macération.

Dans un mode de réalisation préférentiel de l'invention, l'extrait de S. miltiorrhiza, en particulier des parties aériennes, est obtenu par macération d'au moins une partie de la plante, préférentiellement la partie aérienne, en particulier les feuilles, dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol et/ou butylène glycol et/ou autres glycols, tel que xylitol etc.) de 100/0 à 0/100 (p/p). Plus préférentiellement, le solvant utilisé est constitué par de l'eau seulement.
En particulier, l'extrait est obtenu par extraction aqueuse. Au sens de la présente invention, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau.

L'extraction peut être réalisée durant une période allant de 1 heure à 20 heures, préférentiellement de 2 heures à 16 heures. Plus préférentiellement, l'extraction est réalisée durant une période de 2 heures.
L'extraction peut être réalisée à une température comprise entre 0°C à 80 °C, préférentiellement entre 0 °C et 25 °C, plus préférentiellement entre 4 °C et 20 °C. Encore plus préférentiellement, l'extraction est réalisée à température ambiante, c'est-à-dire à 20 °C.

Dans un mode de réalisation de l'invention, la niacine (CAS n°59-67-6) et/ou le niacinamide (CAS n°98-92-0) est préparée par dissolution dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol et/ou butylène glycol et/ou autres glycols, tel que xylitol etc.) de 100/0 à 0/100 (v/v). Plus préférentiellement, le solvant utilisé est constitué par de l'eau seulement.

Dans un mode préférentiel de réalisation de l'invention, la niacine et/ou le niacinamide est ajoutée sous forme de poudre de façon concomitante à la préparation de l'extrait de S. miltiorrhiza. Dans ce cas, la niacine et/ou le niacinamide est ajoutée à une concentration comprise entre 1.10⁻⁷ % et 10%, préférentiellement entre 1.10⁻⁵ % et 5 %, plus préférentiellement entre 1.10⁻¹ % et 3 %, et encore plus préférentiellement à 1,3%, en poids par rapport au poids total de la combinaison (p/p).
Dans un mode alternatif de réalisation de l'invention, la niacine et/ou le niacinamide est ajoutée sous forme de poudre en fin de préparation de l'extrait de Salvia miltiorrhiza, à une concentration comprise entre 1.10⁻⁷ % et 10%, préférentiellement entre 1.10⁻⁵ % et 5 %, plus préférentiellement entre 1.10⁻¹ % et 3 %, et encore plus préférentiellement à 1,3 %, en poids par rapport au poids total de la combinaison (p/p).
Dans un mode de réalisation préférentiel de l'invention, la combinaison selon l'invention est une combinaison de niacine et/ou de niacinamide et d'un extrait de Salvia miltiorrhiza, ledit extrait étant obtenu par extraction dans un solvant, à une concentration comprise entre 1 à 10 %, préférentiellement entre 1% et 5 %, encore préférentiellement entre 2% et 3%, en poids de matière fraîche d'au moins une partie de la plante par rapport au poids total de la partie de la plante et du solvant, en particulier des parties aériennes.
Dans un mode préférentiel de réalisation de l'invention, la combinaison est une combinaison de niacine et/ou de niacinamide et d'un extrait aqueux de Salvia miltiorrhiza pour laquelle ledit extrait est obtenu par macération dans l'eau sous agitation à température ambiante, c'est-à-dire à 20 °C, durant une période de 2 heures, de 2,6 % de feuilles broyées de Salvia miltiorrhiza en poids par rapport au poids total des feuilles et de l'eau, et la niacine et/ou le niacinamide est ajoutée de façon concomitante durant la macération à une concentration finale de 1,3% en poids par rapport au poids total du mélange eau, feuilles et niacine et/ou niacinamide, tel que décrit dans l'exemple 1a).
Dans un autre mode de réalisation de l'invention, la combinaison est une combinaison de niacine et/ou de niacinamide et d'un extrait aqueux de S. miltiorrhiza obtenu par macération dans l'eau sous agitation à 4 °C, durant une période de 16 heures, de 2,6 % de feuilles broyées de S. miltiorrhiza en poids par rapport au poids total des feuilles et de l'eau, la niacine et/ou le niacinamide étant ajoutée de façon concomitante durant la macération à une concentration finale de 1,3% en poids par rapport au poids total du mélange eau, feuilles et niacine et/ou niacinamide, tel que décrit dans l'exemple 1b).
Dans encore un autre mode de réalisation de l'invention, la combinaison est une combinaison de niacine et/ou de niacinamide et d'un extrait de S. miltiorrhiza obtenu par macération sous agitation dans un mélange eau/butylène glycol à 75%/25% (p/p) à température ambiante, c'est-à-dire à 20 °C, durant une période de 2 heures, de 2,6 % de feuilles broyées de S. miltiorrhiza en poids par rapport au poids total des feuilles et du mélange eau/butylène glycol, la niacine et/ou le niacinamide étant ajoutée de façon concomitante durant la macération à une concentration finale de 1,3% en poids par rapport au poids total du mélange eau/butylène glycol, feuilles et niacine et/ou niacinamide, tel que décrit dans l'exemple 1c).
La combinaison peut aussi être une combinaison de niacine et/ou de niacinamide et d'un extrait de S. miltiorrhiza obtenu par macération sous agitation dans l'eau de racines broyées à température ambiante, c'est-à-dire à 20 °C, durant une période de 2 heures, à une concentration finale de 2,6 % de racines en poids par rapport au poids total de l'eau et des racines, la niacine et/ou le niacinamide étant ajoutée de façon concomitante durant la macération à une concentration finale de 1,3% en poids par rapport au poids total du mélange eau, racine et niacine et/ou niacinamide, tel que décrit dans l'exemple 1d).

La combinaison ainsi obtenue est ensuite centrifugée et/ou filtrée afin de récupérer la fraction soluble active (extrait brut), préférentiellement la fraction hydrosoluble. Des étapes supplémentaires de décoloration et/ou désodorisation peuvent être effectuées sur la combinaison à n'importe quel stade de l'extraction et selon les techniques connues de l'Homme du métier. Préférentiellement, la combinaison est filtrée sur grille (250 µm) puis ultrafiltrée.
Ainsi, de façon particulièrement avantageuse, la combinaison selon l'invention est une combinaison d'un extrait de feuilles de Salvia miltiorrhiza, préférentiellement un extrait obtenu par extraction aqueuse, et de niacine et/ou de niacinamide.

L'invention a en outre pour objet l'utilisation d'une combinaison d'un extrait de Salvia miltiorrhiza et de niacine et/ou de niacinamide selon l'invention pour augmenter la déglycation des protéines de la MEC et/ou augmenter l'expression du collagène afin notamment de maintenir et/ou augmenter la fermeté et/ou l'élasticité de la peau et/ou des muqueuses et/ou du cuir chevelu et/ou de prévenir et/ou lutter contre le vieillissement cutané et/ou de maintenir et/ou augmenter l'éclat du teint de la peau. Préférentiellement, la combinaison augmente la déglycation du collagène, préférentiellement du collagène humain, et préférentiellement encore le collagène de type I, en particulier humain.
Au sens de la présente invention, on entend par « maintenir et/ou augmenter la fermeté et/ou l'élasticité », une augmentation à des fins esthétiques de la fermeté et/ou de l'élasticité de la peau et/ou des muqueuses et/ou du cuir chevelu qui ont perdu de la fermeté et/ou de l'élasticité notamment sous l'effet de facteurs intrinsèques tels que le vieillissement tissulaire de la peau et/ou des muqueuses et/ou du cuir chevelu, c'est-à-dire le vieillissement chronoinduit, que l'on retrouve par exemple chez les peaux dites matures c'est-à-dire les peaux des personnes de plus de 40 ans, le stress cellulaire, les variations physiologiques non pathologiques telles qu'un changement de régime alimentaire, les variations hormonales, en particulier durant la puberté, la grossesse, la ménopause, l'andropause. Cette perte de fermeté et/ou d'élasticité peut également apparaitre sous l'effet de facteurs extrinsèques tels que les agents agressifs de l'environnement comme par exemple les radiations UV, la pollution, les fumées, le tabac, les toxines, les agressions climatiques et/ou mécaniques. Il peut notamment s'agir du soin et/ou traitement cosmétique de l'aspect inesthétique et/ou inconfortable des vergetures.
L'augmentation de la fermeté peut être mesurée selon les méthodes classiques notamment par mesure in vivo à l'aide d'un cutomètre, d'un densiscore, d'un torquemètre ou encore d'un dynaskin associé à un dermatop. L'augmentation de l'élasticité peut être mesurée selon les méthodes classiques notamment par mesure in vivo, à l'aide d'un cutomètre, d'un ballistomètre, d'un torquemètre, d'un cornéovacuomètre ou encore d'un densiscore.
L'augmentation de l'élasticité peut notamment être mesurée par les méthodes classiques consistant à mesurer la capacité de la peau à revenir à son état initial après torsion et/ou déformation. La méthode dite de projection à franges permettra par ailleurs de mesurer in vivo les rides. Au sens de la présente invention, on entend par « vieillissement cutané » notamment le vieillissement chronobiologique observé au niveau de la peau des peaux dites matures telles que définies ci-dessus mais aussi le vieillissement photobiologique, c'est à dire induit par les radiations UV. Le vieillissement cutané se manifeste par l'apparition de rides et/ou de ridules, par l'apparition de pattes d'oies mais aussi par une perte de fermeté et/ou d'élasticité telles que définies ci-dessus.
Au sens de la présente invention, on entend par « maintenir et/ou augmenter l'éclat du teint de la peau» maintenir une peau notamment non terne, lumineuse, et/ou diminuer l'aspect jaunâtre de la peau et/ou augmenter la luminosité de la peau, avantageusement par homogénéisation du teint et/ou en lui donnant un aspect lumineux, éclatant, en bonne santé et/ou nourri, un effet bonne mine. En effet, l'éclat du teint traduit un état de bonne santé de la peau. De nombreux facteurs intrinsèques ou extrinsèques peuvent provoquer un teint brouillé, inhomogène, parmi lesquels le stress, la fatigue, les changements hormonaux, la déshydratation de l'épithélium, préférentiellement l'épiderme, les agents polluants et le vieillissement chronobiologique et photobiologique. Ces facteurs tendent à brouiller le teint, le rendre terne, cireux, voire maladif. En particulier, l'aspect jaunâtre de la peau est notamment induit par la présence au niveau du derme de protéines glyquées.
La mesure de l'éclat du teint et/ou la luminosité de la peau peut par exemple être mesurée par chromamétrie ou par analyse d'image. Cette dernière méthode de mesure in vivo consiste à prendre des photographies haute résolution en configuration polarisée croisées du visage de volontaires prises à 45° avant et après application du produit testé. Sur la base de ces photographies numériques, une analyse d'image permet d'extraire et de quantifier des paramètres spécifiques (par exemple: L*, a*, b*, C, h°) reliés à la couleur, l'éclat, l'homogénéité, et la texture de la peau. Au sens de la présente invention, on entend par « muqueuse(s) », la muqueuse oculaire, la muqueuse vaginale, la muqueuse uro-génitale et/ou la muqueuse buccale, notamment la muqueuse buccale labiale et/ou la muqueuse gingivale, préférentiellement, les muqueuses oculaires et/ou buccales, et encore préférentiellement, la muqueuse labiale et/ou oculaire. Dans un mode de réalisation particulier la combinaison selon l'invention est appliquée par voie topique sur des parties spécifiques du corps choisies parmi le cou, le décolleté, le ventre, les bras, les cuisses, les hanches, les fesses, la taille et/ou le visage, en particulier le contour des yeux et/ou des lèvres, le front et/ou les zones temporales du cuir chevelu, en particulier pour les utilisations définies ci-dessus.

La combinaison de l'extrait de S. miltiorrhiza et de niacine et/ou de niacinamide selon l'invention est une combinaison cosmétique et/ou pharmaceutique, notamment dermatologique, et/ou nutraceutique. Elle peut être administrée par voie orale, notamment sous forme de gélules, ou sous forme topique. Elle est oralement et/ou topiquement acceptable.
Au sens de la présente invention, on entend par « oralement et/ou topiquement acceptable », une combinaison adaptée à une application par voie orale et/ou topique, non toxique, non irritant pour la peau et/ou les muqueuses et/ou le cuir chevelu, n'induisant pas de réponse allergique, qui n'est pas instable sur le plan chimique.
Au sens de la présente invention, on entend par « application par voie topique » d'un ingrédient et/ou d'une combinaison, l'application locale directe et/ou la vaporisation de l'ingrédient et/ou d'une combinaison sur la surface de la peau et/ou des muqueuses et/ou le cuir chevelu.
Au sens de la présente invention, on entend par « augmenter la déglycation des protéines de la MEC » au niveau de la peau et/ou des muqueuses et/ou du cuir chevelu, diminuer la quantité de protéines glyquées au niveau de la MEC, qui peut notamment être mesurée par la diminution de la quantité de carboxyméthyllysine (CML), marqueur connu de la synthèse des AGEs, formée lorsqu'une protéine de la MEC préalablement glyquée par l'intermédiaire d'un sucre réducteur est mise en présence de la combinaison selon l'invention. On entend par « sucre réducteur » le ribose, glucose, fructose, xylose et leurs métabolites, tel que le glyoxal, le methylglyoxal, préférentiellement, le ribose.
Au sens de la présente invention, on entend par « protéines de la MEC » le collagène, l'élastine, la fibuline, la fibronectine et la laminine, préférentiellement, le collagène et l'élastine, encore préférentiellement le collagène, plus préférentiellement le collagène humain, et encore plus préférentiellement le collagène de type I, en particulier humain.
Au sens de la présente invention, on entend par collagène le collagène de type I et/ou III et/ou IV et/ou V et/ou VI et/ou VII et/ou XII et/ou XIII et/ou XIV et/ou XVI et/ou XVII et/ou XXIV et/ou XXIX, en particulier présent dans la peau et/ou les muqueuses et/ou le cuir chevelu. Préférentiellement, il s'agit du collagène de type I et/ou III et/ou V, en particulier présent dans la peau et/ou les muqueuses et/ou le cuir chevelu, et plus préférentiellement du collagène de type I, en particulier présent dans la peau et/ou les muqueuses et/ou le cuir chevelu.
Au sens de la présente invention, la combinaison selon l'invention est considérée comme étant en quantité efficace pour augmenter la déglycation du collagène lorsque la quantité de CML mesurée en présence du collagène humain de type I glyqué avec du ribose, dans les conditions décrites selon l'exemple 2, est diminuée d'au moins 15 %, plus préférentiellement d'au moins 20 %, encore plus préférentiellement d'au moins 25 %, par rapport à la quantité de CML mesurée lorsque ledit collagène glyqué avec ribose est incubé sans combinaison.
La combinaison de l'extrait de Salvia miltiorrhiza et de niacine et/ou de niacinamide, présente un effet de synergie sur la déglycation du collagène de type I humain. Au sens de la présente invention, l'effet de synergie correspond à une diminution de la quantité de CML mesurée en présence de la combinaison de l'extrait de Salvia miltiorrhiza et de niacine et/ou de niacinamide, d'au moins 2 %, préférentiellement d'au moins 5 %, encore plus préférentiellement d'au moins 10 % de plus que lorsque l'extrait de Salvia miltiorrhiza est incubé seul sans niacine et sans niacinamide et lorsque la niacine et/ou le niacinamide en solution est incubée seule dans les mêmes conditions, en présence de collagène humain de type I glyqué avec du ribose sans l'extrait de Salvia miltiorrhiza, selon les conditions décrites dans l'exemple 2.

Au sens de la présente invention, on entend par « augmenter l'expression du collagène » l'augmentation de l'expression génique, c'est-à-dire l'augmentation des ARN messagers, et/ou l'augmentation protéique du collagène, préférentiellement l'augmentation protéique du collagène, et plus préférentiellement du collagène de type I, en particulier au niveau de la peau et/ou des muqueuses et/ou du cuir chevelu.
Au sens de la présente invention, la combinaison selon l'invention est considérée comme étant en quantité efficace pour augmenter l'expression du collagène lorsque l'augmentation de l'expression du collagène est d'au moins 5%, plus préférentiellement d'au moins 20%, encore plus préférentiellement d'au moins 40 %, par rapport au niveau d'expression mesuré en l'absence de la combinaison selon l'invention. Préférentiellement, il s'agit de l'augmentation de l'expression protéique du collagène de type I humain obtenu à partir de fibroblastes issus de biopsies abdominales, mesurée selon le protocole de l'exemple 3 en présence de la combinaison de l'extrait de feuilles de Salvia miltiorrhiza et de niacine préparée selon l'exemple 1a.
La combinaison de l'extrait de Salvia miltiorrhiza et de niacine et/ou de niacinamide, présente en outre aussi un effet de synergie sur l'augmentation de l'expression protéique de collagène. On entend ici par « effet de synergie sur l'augmentation de l'expression protéique du collagène » une augmentation de l'expression protéique du collagène de type I humain, d'au moins 20%, préférentiellement d'au moins 25% de plus que le niveau d'expression protéique dudit collagène mesuré selon l'exemple 3 en présence d'un extrait de Salvia miltiorrhiza seul, c'est-à-dire sans niacine et sans niacinamide, et mesuré en présence de niacine et/ou de niacinamide seul sans extrait de Salvia miltiorrhiza.

Dans un mode de réalisation de l'invention, la combinaison selon l'invention est considérée comme étant en quantité efficace pour augmenter l'éclat du teint de la peau lorsque la diminution du jaunissement de la peau, est d'au moins 3%, préférentiellement d'au moins 5% au bout de 112 jours par rapport au temps 0 d'application d'une crème contenant la combinaison selon l'invention. Dans un mode préférentiel de réalisation de l'invention, cette diminution du jaunissement de la peau est mesurée en évaluant la diminution de la fluorescence de la peau à une longueur d'onde de 370 nm (exc)/475 nm (em) mesurée par chromamétrie (paramètre b*) sur l'hémivisage de femmes caucasiennes dans les conditions exposées dans l'exemple 5. Dans un mode alternatif de réalisation de l'invention, la diminution de la fluorescence de la peau est évaluée par analyse d'image. Préférentiellement alors, la diminution de la fluorescence est alors d'au moins 5%, préférentiellement d'au moins 8% au bout de 118 jours par rapport au temps 0 d'application d'une crème contenant la combinaison selon l'invention, conformément aux conditions exposées dans l'exemple 5.

Un objet de la présente invention concerne donc l'utilisation cosmétique et/ou nutraceutique de la combinaison selon l'invention.
Au sens de la présente invention, on entend par « utilisation cosmétique et/ou nutraceutique » une utilisation non thérapeutique, c'est-à-dire qui n'est pas destinée à un usage thérapeutique pour traitement. L'utilisation selon la présente invention vise donc des parties du corps et/ou des cellules saines. On entend par « des parties du corps saines » des parties de corps « normales », c'est-à-dire qualifiées de non pathologiques par un dermatologue et/ou un médecin, qui ne nécessitent pas de traitement thérapeutique, qui ne présentent pas d'infection, de maladie ou affection cutanée telle que candidose, impétigo, psoriasis, eczéma, acné ou dermatite. On entend par « cellule saine » une cellule « normale », c'est-à-dire non pathologique, notamment non cancéreuse et qui ne nécessite pas de traitement thérapeutique.

Ainsi, la combinaison de l'extrait de Salvia miltiorrhiza et de niacine et/ou de niacinamide peut être utilisée seule sous forme d'ingrédient actif ou dans une composition cosmétique et/ou pharmaceutique, notamment dermatologique, et/ou nutraceutique.
Lorsqu'elle est utilisée sous forme d'ingrédient actif, la combinaison selon l'invention est préférentiellement soluble et dissoute dans un solvant notamment polaire, tel que l'eau, un alcool, un polyol, un glycol, ou un de leurs mélanges, préférentiellement un mélange hydroglycolique, encore préférentiellement contenant un glycol choisi parmi le caprylyl glycol, l'hexylène glycol, le pentylène glycol et leurs mélanges. Avantageusement, la combinaison selon l'invention est dissoute dans une solution aqueuse contenant de l'hexylène glycol et du caprylyl glycol, en particulier contenant entre 0,01 et 10 % en poids de l'hexylène glycol et entre 0,01 et 10 % en poids de caprylyl glycol par rapport au poids total la solution aqueuse, plus particulièrement entre 0,5 % et 5 % en poids d'hexylène glycol et entre 0,1% et 1% en poids de caprylyl glycol par rapport au poids total la solution aqueuse. Préférentiellement, la concentration en caprylyl glycol est de 1 % en poids par rapport au poids total la solution aqueuse et la concentration en hexylène glycol est de 0,5 % en poids par rapport au poids total la solution aqueuse. Préférentiellement, l'ingrédient actif selon la présente invention contient aussi de la gomme de xanthane à une concentration comprise entre 0,01 et 10 %, préférentiellement entre 0,1 % et 1 %, encore préférentiellement 0,5%, en poids par rapport au poids total de la solution aqueuse, selon l'exemple 6.
Dans un mode de réalisation avantageux, la combinaison selon l'invention est solubilisée ou dissoute dans une solution aqueuse contenant en outre de l'hexylene glycol, préférentiellement en une teneur de 0,5%, du caprylyl glycol, préférentiellement en une teneur de 1%, et de la gomme de xanthane, préférentiellement en une teneur de 0,5%, en poids par rapport au poids total de la solution aqueuse.
Dans un mode particulier de réalisation de l'invention, l'ingrédient actif comprenant la combinaison de l'extrait de S. miltiorrhiza et de niacine et/ou de niacinamide est stérilisé.
Préférentiellement, l'ingrédient actif est présent dans une composition cosmétique et/ou pharmaceutique, préférentiellement dermatologique, et/ou nutraceutique à une concentration comprise entre 3 % et 5 %, encore préférentiellement de 3 %, en poids par rapport au poids total de la composition.
De façon particulièrement avantageuse, l'extrait de S. miltiorrhiza selon l'invention, préférentiellement obtenu par extraction aqueuse, et la niacine et/ou le niacinamide sont présents dans l'ingrédient actif tel que défini ci-dessus à une concentration finale entre 0,25% et 5% d'extrait de S. miltiorrhiza, préférentiellement 0,25%, et entre 1% et 5%, préférentiellement 1,2 % de niacine, en poids par rapport au poids total de l'ingrédient actif.

Un autre objet de l'invention concerne encore une composition cosmétique et/ou nutraceutique comprenant la combinaison selon l'invention, éventuellement sous forme d'ingrédient actif, et au moins un excipient cosmétiquement et/ou nutraceutiquement acceptable.
Dans un mode de réalisation de l'invention, la composition est une composition nutraceutique, administrable par voie orale, comprenant la combinaison selon l'invention. Au sens de la présente invention, on entend par « composition nutraceutique » une composition administrable en tant que complément alimentaire, non thérapeutique. La composition nutraceutique contient par ailleurs au moins un excipient nutraceutiquement acceptable, c'est-à-dire, administrable par voie orale tel que déjà défini dans la présente invention.

Dans un mode préférentiel de réalisation de l'invention, la composition est une composition cosmétique, préférentiellement applicable par voie topique.
L'extrait de S. miltiorrhizae selon l'invention est ainsi présent dans la composition cosmétique et/ou nutraceutique à une concentration finale comprise entre 1.10⁻⁴ % et 10 % en poids par rapport au poids total de la composition, avantageusement entre 1.10⁻⁴ % et 5 %, plus préférentiellement entre 1.10⁻³ % et 5 %, et de façon particulière entre 0,01 % et 5 % en poids par rapport au poids total de la composition.
La niacine et/ou le niacinamide est présente dans la composition cosmétique et/ou nutraceutique à une concentration finale comprise entre 1.10⁻⁷ % et 10 % en poids par rapport au poids total de la composition, avantageusement entre 1.10⁻⁴ % et 5 %, plus préférentiellement entre 1.10⁻³ % et 5 %, et de façon particulière entre 0,01 % et 5 % en poids par rapport au poids total de la composition.

La composition cosmétique comprend en outre au moins un excipient cosmétiquement acceptable. Avantageusement, le ou lesdits excipients sont choisis parmi au moins un des groupes constitués par les conservateurs, les émollients, les émulsifiants, les tensioactifs, les hydratants, les épaississants, les agents de texture, les agents filmogènes, les pigments, les stabilisants, les solubilisants, les colorants, les parfums.
Avantageusement encore, le ou les excipients sont choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les gommes de xanthane, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de cœur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée. La composition de l'invention peut être choisie parmi une solution, aqueuse ou huileuse, une crème ou un gel aqueux ou un gel huileux, notamment un gel douche, un shampoing, un lait, une émulsion, une microémulsion ou une nanoémulsion, notamment huile-dans-eau ou eau-dans-huile ou multiple ou siliconée, un masque, un sérum, une lotion, un savon liquide, un pain dermatologique, une pommade, une mousse, un patch, un produit anhydre, de préférence liquide, pâteux ou solide, par exemple sous forme de poudres de maquillage, de bâtonnet ou de stick, notamment sous forme de rouge à lèvre. Préférentiellement, la composition cosmétique selon l'invention est une crème ou un sérum.
De façon avantageuse, la composition cosmétique selon l'invention est une crème ou un sérum, destinée à être appliquée sur des parties spécifiques du corps choisies parmi le cou, le décolleté, ventre, les bras, les cuisses, les hanches, les fesses, la taille et/ou le visage, en particulier le contour des yeux et/ou des lèvres, le front et/ou les zones temporales du cuir chevelu, et préférentiellement les sillons nasogéniens, la zone périorbitaire, en particulier les cernes et les pattes d'oies.
En outre, la composition cosmétique et/ou nutraceutique de la présente invention peut contenir un ou plusieurs autres ingrédients actifs cosmétiques et/ou nutraceutiques, conduisant à un effet complémentaire et/ou un effet de synergie avec la combinaison selon l'invention.
Il peut s'agir par exemple d'ingrédients cosmétiques actifs « déglyquants » et/ou « antiglycation » tels qu'un extrait de Davilla rugosa commercialisé sous le nom de Collguard™ par BASF Beauty Care Solutions ; un extrait de feuilles de la plante Manilkara multinervisun commercialisé sous le nom d'Elestan™ ; extrait de Rosmarinus officinalis, en particulier un extrait de feuilles, par exemple commercialisé sous forme de gélules ; un extrait de feuille d'Argania spinosa commercialisé sous le nom d'Arganyl™ par la demanderesse, un extrait de plante du genre hemerocallis, en particulier de fleur ; un autre extrait de plante du genre Salvia, en particulier des espèces Salvia officinalis, Salvia sclarea ou Salvia pratensis ; un extrait de Helianthus annuus, en particulier de graines ou encore un extrait de l'algue Hypnea musciformis. On entend ici par « ingrédients actifs antiglycation » des ingrédients actifs capables d'inhiber la réaction de glycation, c'est-à-dire d'inhiber la formation des AGEs.
Il peut s'agir par ailleurs d'ingrédients actifs anti-âges et/ou agents tenseurs pour un effet de synergie avec l'ingrédient actif de l'invention. Avantageusement, il s'agit d'ingrédients actifs augmentant l'expression génique et/ou protéique du collagène et/ou empêchant la dégradation dudit collagène tels que le rétinol, la vitamine C, un extrait de Quassia amara ou un extrait de Davilla rugosa commercialisé sous le nom de Collguard™ par BASF Beauty Care Solutions, un extrait de graine d'Hibiscus abelmoschus commercialisé sous le nom de Linefactor™, un peptide commercialisé sous le nom de Dermican™ par la demanderesse ou encore les produits commercialisés sous les noms de Matrixyl™, Matrixyl 3000™ et Regestril™ par la société Sederma.
Il peut aussi s'agir d'actifs anti-rides pour maintenir et/ou augmenter l'élasticité de la peau tel qu'un extrait d'aneth commercialisé sous le nom de Lyslastine™ par la demanderesse.
Enfin, il peut s'agir d'agents stimulant l'activité ou la prolifération des fibroblastes, tel qu'un extrait de protéine de soja modifiée commercialisé sous le nom de Phytokine™ par la demanderesse.
Les agents tenseurs utilisables dans l'invention peuvent être aussi choisis parmi les polymères synthétiques, tels que les latex de polyuréthanne ou les latex acryliques, les polymères d'origine naturelle, notamment les polyholosides sous forme d'amidon ou sous forme de carraghénanes, alginates, agars, gellanes, polymères cellulosiques et pectines, les protéines et hydrolysats de protéines végétales de soja, les silicates mixtes, les microparticules de cire, les particules colloïdales de charge inorganique choisies par exemple parmi la silice, les composites silice-alumine ; ainsi que leurs mélanges. Il peut enfin s'agir d'ingrédients cosmétiques et/ou dermatologiques comme par exemple des agents antimicrobiens, agents anti-radicalaires, agents apaisants, calmants ou relaxants, agents agissant sur la microcirculation pour augmenter l'éclat du teint, en particulier du visage, agents cicatrisants.
Parmi les agents antimicrobiens pouvant être associés à l'ingrédient actif de l'invention dans la présente invention, on peut citer le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, le farnesol, les phytosphingosines et leurs mélanges. Les agents anti-radicalaires peuvent être la vitamine C et ses dérivés dont le glucoside d'ascorbyle, les phénols et polyphénols, en particulier les tannins, l'acide ellagique et l'acide tannique; l'épigallocatéchine et les extraits naturels en contenant, en particulier les extraits de thé vert, les anthocyanes, les acides phénols, les stilbènes, des actifs piégeurs de composés aromatiques mono- ou polycycliques les tannins tels que l'acide ellagique et les dérivés indoles et/ou des actifs piégeurs de métaux lourds tels que l'EDTA, des actifs anti-radicalaires tels que la vitamine E et ses dérivés tels que l'acétate de tocophéryle, les bioflavonoïdes, la coenzyme Q10 ou ubiquinone.
La composition cosmétique et/ou nutraceutique de la présente invention peut aussi contenir un ou plusieurs agents anti-radicalaires et/ou antioxydants tel qu'un extrait de racine de Rhodiola crenulata commercialisé sous le nom de Rhodiomax™ par la demanderesse.
Comme agents apaisants entrant dans la composition de l'invention, on peut utiliser les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de Pueraria lobata commercialisé sous le nom Inhipase™ par la demanderesse, les extraits de Theobroma cacao. Les ingrédients actifs agissant sur la microcirculation, vasoprotecteurs ou vasodilatateurs, peuvent être choisis parmi les flavonoïdes, les ruscogénines, les nicotinates, les huiles essentielles.

L'invention concerne de plus l'utilisation de la composition cosmétique et/ou nutraceutique selon l'invention pour augmenter la déglycation du collagène et/ou augmenter l'expression du collagène pour maintenir et/ou augmenter la fermeté et/ou l'élasticité de la peau et/ou des muqueuses et/ou du cuir chevelu, et/ou pour prévenir et/ou lutter contre le vieillissement cutané et/ou pour maintenir et/ou augmenter l'éclat du teint de la peau.
En particulier le collagène est le collagène de type I.
De façon avantageuse la composition est appliquée par voie topique sur des parties spécifiques du corps choisies parmi le cou, le décolleté, le ventre, les bras, les cuisses, les hanches, les fesses, la taille et/ou le visage, en particulier le contour des yeux et/ou des lèvres, le front et/ou les zones temporales du cuir chevelu.

L'invention concerne en outre un procédé de soin cosmétique comprenant l'application, préférentiellement par voie topique, de la combinaison selon l'invention ou de la composition cosmétique ou nutraceutique selon l'invention. Dans un mode préférentiel de réalisation de l'invention, le procédé de soin cosmétique comprend l'application sur une zone de peau et/ou de muqueuse et/ou de cuir chevelu, préférentiellement par voie topique, de la combinaison selon l'invention ou d'une composition selon l'invention comprenant la combinaison selon l'invention pour augmenter la déglycation du collagène et/ou augmenter l'expression du collagène afin notamment de maintenir et/ou augmenter la fermeté et/ou l'élasticité de la peau et/ou des muqueuses et/ou du cuir chevelu et/ou de prévenir et/ou lutter contre le vieillissement cutané et/ou de maintenir et/ou augmenter l'éclat du teint de la peau.
Préférentiellement, le procédé de soin cosmétique est caractérisé en ce que la zone de peau et/ou de muqueuse et/ou de cuir chevelu est choisie au moins parmi le cou, le décolleté, ventre, les bras, les cuisses, les hanches, les fesses, la taille et/ou le visage, en particulier le contour des yeux et/ou des lèvres, le front et/ou les zones temporales du cuir chevelu, et préférentiellement les sillons nasogéniens, la zone périorbitaire, en particulier les cernes et les pattes d'oies. Préférentiellement encore, le procédé de soin cosmétique selon l'invention est caractérisé en ce que la zone de peau et/ou de muqueuse et/ou de cuir chevelu est une zone saine, c'est-à-dire consistant essentiellement en une zone constituée de cellules saines telles que précédemment définies dans la présente invention. Préférentiellement, la population d'individus visée par le procédé de soin cosmétique est une population « normale », c'est-à-dire saine, qui ne nécessite pas de traitement thérapeutique, de façon particulière de traitement contre le cancer.
L'invention concerne enfin la combinaison d'un extrait de Salvia miltiorrhiza et de niacine et/ou de niacinamide selon l'invention en tant qu'ingrédient actif, ainsi qu'une composition pharmaceutique, notamment dermatologique, la comprenant pour son utilisation, préférentiellement par voie topique, pour augmenter la déglycation du collagène et/ou augmenter l'expression du collagène, préférentiellement le collagène de type I pour prévenir et/ou lutter contre les pathologies impliquant une perte de fermeté et/ou d'élasticité de la peau et/ou des muqueuses et/ou du cuir chevelu, notamment telles que la couperose, les télangiectasies, l'élastose solaire, la maladie cutix laxa et/ou les vergetures. Un dernier objet de l'invention concerne une composition pharmaceutique, préférentiellement dermatologique, et encore préférentiellement applicable par voie topique, comprenant la combinaison selon l'invention pour augmenter la cicatrisation de la peau et/ou des muqueuses et/ou du cuir chevelu.

Des exemples faisant référence à la description de l'invention sont présentés ci-après. Ces exemples sont donnés à titre d'illustration et ne sauraient limiter en aucun cas la portée de l'invention. Chacun des exemples a une portée générale.
Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

### Exemple 1 : Préparation de différentes combinaisons d'extrait de Salvia miltiorrhiza et/ou de niacine selon l'invention

### Exemple 1a)

Des feuilles de Salvia miltiorrhiza ont été broyées puis mises à macérer sous agitation dans de l'eau à température ambiante, ici 20 °C, durant une période de 2 heures, à une teneur finale de 2,6 % en poids de matière fraiche par rapport au poids total des feuilles, de la niacine et de l'eau. Un ajout de niacine sous forme de poudre, concomitant à l'étape de macération a été effectué, la concentration finale en niacine étant de 1,3 % en poids par rapport au poids total des feuilles, de la niacine et de l'eau. La combinaison ainsi obtenue a ensuite été filtrée sur grille (250 µm) puis ultrafiltrée.

L'extrait aqueux de feuilles de Salvia miltiorrhiza obtenu dans les conditions décrites ci-dessus, sans ajout concomitant de niacine d'une part, la niacine telle qu'obtenue ci-dessus, sans extrait de feuilles de Salvia miltiorrhiza d'autre part, ainsi que la combinaison décrite ci-dessus ont été utilisés pour tester leur activité de déglycation (Exemple 2).

### Exemple 1b)

Des feuilles de Salvia miltiorrhiza ont été broyées puis mises à macérer sous agitation dans de l'eau à une température de 4 °C, durant une période de 16 heures, à une concentration finale de 2,6 % en poids de matière fraiche par rapport au poids total des feuilles, de la niacine et de l'eau. Un ajout de niacine concomitant à l'étape de macération a été effectué, la concentration finale en niacine étant de 1,3 % en poids par rapport au poids total des feuilles, de la niacine et de l'eau. La combinaison ainsi obtenue a ensuite été filtrée sur grille (250 µm) puis ultrafiltrée.

### Exemple 1c)

Des feuilles de Salvia miltiorrhiza ont été broyées puis mises à macérer dans un mélange eau/butylène glycol (75/25 ; (v/v)) sous agitation à température ambiante, ici 20 °C, durant une période de 2 heures, à une concentration finale de 2,6 % en poids de matière fraiche par rapport au poids total des feuilles, de la niacine et du mélange de solvant.
Un ajout de niacine concomitant à l'étape de macération a été effectué à une concentration finale de 1,3 % en poids par rapport au poids total des feuilles, de la niacine et du mélange de solvant. La combinaison ainsi obtenue a ensuite été filtrée sur grille (250 µm) puis ultrafiltrée.

Exemple 1d) (exemple de référence qui ne fait pas partie de l'invention)Des racines de Salvia miltiorrhiza ont été broyées puis mises à macérer sous agitation dans l'eau à température ambiante, ici 20 °C, durant une période de 2 heures, à une concentration finale de 2,6 % en poids de matière fraiche par rapport au poids total des racines de Salvia miltiorrhiza, de la niacine et de l'eau (p/p). Un ajout de niacine concomitant à l'étape de macération a été effectué à une concentration finale de 1,3 % en poids par rapport au poids total des racines de Salvia miltiorrhiza, de la niacine et de l'eau (p/p). La combinaison ainsi obtenue a ensuite été filtrée sur grille (250 µm) puis ultrafiltrée.

### Exemple 2 : Mise en évidence de l'activité déglycation de la combinaison Salvia miltiorrhiza et niacine selon l'invention.

Méthode : L'activité déglycation des échantillons testés a été mesurée par méthode immunoenzymatique (ELISA) à partir de la quantité de carboxyméthyllysine (CML) détectée en présence de collagène humain de type I glyqué avec du ribose, comme décrit ci-après. Du collagène humain de type I a été extrait à partir d'une biopsie issue de chirurgie abdominale d'un donneur sain puis a été glyqué in tubo à 30°C avec du ribose (1M). L'extrait aqueux de feuilles de S. miltiorrhiza préparé dans les conditions et la concentration présentés en exemple 1a) mais sans ajout de niacine et dilué dans du tampon PBS (Phosphate Buffer Saline) pour obtenir les concentrations finales dans le milieu présentées dans le tableau 1 (% p/p) ou de la niacine à partir de poudre diluée dans du tampon PBS à différentes concentrations finales (p/p) ou la combinaison de l'extrait aqueux de feuilles de S. miltiorrhiza et de niacine obtenue selon l'exemple 1a) diluée dans du tampon PBS pour obtenir les concentrations finales dans le milieu présentées dans le tableau 1 (% p/p), ont été incubés durant 24 heures à une température de 30°C avec le collagène glyqué. Un contrôle négatif a été préparé de la même manière en incubant dans les mêmes conditions le collagène préalablement glyqué avec du tampon PBS seul, sans produit à tester. Le mélange a ensuite été déposé sur microplaque de 96 puits en triplicat (n=3). Un anticorps primaire anti-CML a été ajouté à la solution puis la solution a été incubée à 25 °C sous agitation. Après lavage avec un tampon PBS, un anticorps secondaire anti-collagène a été ajouté au milieu puis le milieu a été incubé à nouveau à 25 °C. Les milieux ont ensuite été incubés sous agitation à l'obscurité durant 8 minutes à 25°C. Une solution contenant de l'acide sulfurique (H₂SO₄) a été ajoutée au milieu pour stopper la réaction puis la densité optique a été lue au spectrophotomètre à 450 nm.

Résultats : Plusieurs concentrations en niacine seule ont été testées, de même que plusieurs concentrations en extrait de S. miltiorrhiza préparé selon l'exemple 1a) seul. Enfin, la combinaison préparée selon l'exemple 1a) a été testée aux concentrations présentées dans le tableau 1. Les résultats sont exprimés en quantité (µg/mL) de CML moyenne mesurée dans les échantillons, ainsi qu'en pourcentage de CML moyenne présente par rapport au pourcentage de CML présente dans le contrôle négatif.

**Tableau 1.**

| Echantillon | CML (µg/mL) | % de CML versus contrôle négatif |
|---|---|---|
| Contrôle négatif (-) | 0,38 | 100 |
| Niacine 1,2.10⁻³ % (p/p) | 0,356 | 94 |
| Niacine 3.10⁻³ % (p/p) | 0,344 | 90,52 |
| Niacine 6.10⁻³ % (p/p) | 0,36 | 95 |
| Extrait Salvia miltiorrhiza (Ex. 1a) 0,10% (p/p) | 0,36 | 95 |
| Extrait Salvia miltiorrhiza (Ex. 1a) 0,25 % (p/p) | 0,368 | 96,8 |
| Extrait Salvia miltiorrhiza (Ex. 1a) 0,50 % (p/p) | 0,35 | 92 |
| Extrait Salvia miltiorrhiza (Ex. 1a) 0,10% (p/p) et niacine 1,2.10⁻³ % (p/p) | 0,316* | 83* |
| Extrait Salvia miltiorrhiza (Ex. 1a) 0,25 % (p/p) et niacine 3.10⁻³ % (p/p) | 0,31* | 82* |
| Extrait Salvia miltiorrhiza (Ex. 1a) 0,50 % (p/p) et niacine 6.10⁻³ % (p/p) | 0,28** | 74** |

| | | |
|---|---|---|
| (One way annova Dunn test: * pvalue < 0,05 ; *** p < 0,01) | | |

Conclusion : le tableau 1 a montré que la niacine seule en solution ne permet pas de réduire la concentration en CML présente. L'extrait de feuilles de Salvia miltiorrhiza préparé selon l'exemple 1a) seul ne permet pas non plus de réduire la concentration en CML présente, quelle que soit la concentration en extrait de plante. Toutefois, la combinaison de l'extrait aqueux de feuilles de Salvia miltiorrhiza et de niacine préparé selon l'exemple 1a) permet de réduire significativement la concentration en CML présente, ce qui démontre l'effet de synergie de la combinaison selon l'invention.

### Exemple 3 : Augmentation de l'expression de collagène de type I en présence de la combinaison selon l'invention.

### Méthode :

Les conditions expérimentales de mise en évidence de l'augmentation de l'expression protéique du collagène de type I sont celles exposées dans l'exemple 1 de la demande internationale publiée sous le numéro WO2012/175454.

Des fibroblastes humains normaux, c'est-à-dire non cancéreux, obtenus à partir de biopsies abdominales d'un donneur sain ont été ensemencés en plaque 96 puits et cultivés dans un milieu défini (FGM) jusqu'à 100% de confluence.

L'extrait aqueux de feuilles de S. miltiorrhiza préparé dans les conditions et la concentration présentés en exemple 1a) mais sans ajout de niacine et dilué dans du tampon PBS pour obtenir les concentrations finales dans le milieu présentées dans le tableau 2 (% p/p) ou de la niacine préparée à partir de poudre et diluée dans du tampon PBS à différentes concentrations finales (p/p) ou la combinaison de l'extrait aqueux de feuilles de S. miltiorrhiza et de niacine obtenue selon l'exemple 1a) diluée dans du tampon PBS pour obtenir les concentrations finales dans le milieu présentées dans le tableau 2 (% p/p), ont alors été ajoutés dans le milieu de culture. Le même milieu de culture sans ajout d'extrait, de niacine ou de la combinaison a été utilisé comme témoin (Contrôle négatif). Le même milieu avec ajout d'une solution de vitamine C (50 µM) a été utilisé comme contrôle positif.

Après 48 heures de culture post confluence à 37°C, le milieu de culture a été prélevé et éliminé. Une étape de lyse des cellules a été effectuée puis le lysat a été prélevé et analysé. Un dosage d'ADN a été réalisé sur les lysats de façon à exprimer l'expression protéique du collagène par cellule. L'ADN double brin a été dosé par la méthode au bisbenzimide (Invitrogen, Quant-iT™ PicoGreen ® dsDNA). La concentration en ADN est proportionnelle au nombre de cellules viables et permet de rationaliser la fluorescence lue par un nombre de cellules.

Le dosage du collagène de type I a été effectué par technique immunochimique comme suit : un anticorps anti-collagène de type I a été incubé durant 30 minutes avec le lysat cellulaire. Après rinçage au PBS, l'anticorps secondaire couplé à l'europium (Perkin Elmer) a été ajouté. Une solution de révélation a été ajoutée et la fluorescence a été mesurée en utilisant un lecteur multiplaque EnVision (Perkin Elmer).

Pour chaque condition, la fluorescence en temps retardée (TRF) a été mesurée dans chaque puits et rationalisée par la quantité d'ADN dosée dans le puits. Le ratio (fluorescence/concentration d'ADN) a été calculé. Pour chaque condition, les résultats sont exprimés en pourcentage d'expression protéique moyenne par rapport à l'expression protéique moyenne mesurée dans le contrôle négatif.

### Résultats :

**Tableau 2**

| | Stimulation collagène I (% versus non traité) |
|---|---|
| Contrôle négatif (non traité) | 100 |
| Contrôle positif (vit C) (50 µM) | 119 |
| Niacine 1,2.10⁻³ % (p/p) | 95,96 |
| Niacine 3.10⁻³ % (p/p) | 95,14 |
| Niacine 6.10⁻³ % (p/p) | 104,55 |
| Extrait Salvia miltiorrhiza 0,10 % (p/p) | 106,43 |
| Extrait Salvia miltiorrhiza 0,25 % (p/p) | 113,19 |
| Extrait Salvia miltiorrhiza 0,50 % (p/p) | 131,19 * |
| Extrait Salvia miltiorrhiza 0,10 % (p/p) et niacine 1,2.10⁻³ % (p/p) | 116,50 * |
| Extrait Salvia miltiorrhiza (Ex. 1a) 0,25 % (p/p) et niacine 3.10⁻³ % (p/p) | 123,57 * |
| Extrait Salvia miltiorrhiza (Ex. 1a) 0,50 % (p/p) et niacine 6.10⁻³ % (p/p) | 161,38 *** |

| | |
|---|---|
| (One way annova, Dunnet method: * pvalue< 0.05 ; ** pvalue <0,01 ; *** p<0.001) | |

Conclusion: le tableau 2 a montré une augmentation de l'expression protéique moyenne mesurée plus importante en présence de la combinaison selon l'invention qu'en présence de niacine seule d'une part ou de l'extrait de S. miltiorrhiza seul d'autre part, démontrant l'effet de synergie de la combinaison sur l'expression protéique du collagène de type I.

### Exemple 4 : Mise en évidence in vivo de l'activité déglycation sur le collagène glyqué de la combinaison selon l'invention.

Méthode : La mesure in vivo a été effectuée sur la peau du visage d'un échantillon de 27 femmes caucasiennes âgées de 45 ans ou plus, ladite peau étant saine, c'est-à-dire constituée de cellules « normales », non pathologiques, ne nécessitant pas de traitement thérapeutique. La mesure a été effectuée comme suit : une crème préparée selon la formulation 7f) (Exemple 7) et contenant 3 % en poids de l'ingrédient actif selon l'exemple 6 par rapport au poids total de la formulation (p/p), l'ingrédient actif contenant 0,25 % en poids de l'extrait de feuilles de Salvia miltiorrhiza et 1,2 % en poids de niacine, par rapport au poids total de l'ingrédient actif, , a été appliquée sur une moitié de visage 2 fois par jour tous les jours durant 4 mois. Une seconde crème placebo de même composition mais sans la combinaison selon l'invention a été appliquée dans les mêmes conditions sur la seconde moitié de visage.

Une mesure de la quantité de collagène glyqué a été effectuée aux temps 0, 28 jours, 56, 84 et 112 jours par mesure de la fluorescence de la peau à une longueur d'onde de 370 nm (exc)/475 nm (em). Les valeurs ont été normalisées par rapport à la fluorescence mesurée à 396 nm, traduisant l'autofluorescence de la peau.

Résultats : les résultats sont exprimés en pourcentage de diminution moyenne de la fluorescence mesurée sur l'hémivisage des 27 femmes traités avec la crème contenant la combinaison selon l'invention, et pour le contrôle traité avec la crème ne contenant pas ladite combinaison (hémivisage non traité) (n= 27).

**Tableau 3**

| Temps (jours) | Contrôle (%) | Combinaison (%) |
|---|---|---|
| 0 | 100 | 100 |
| 28 | 102 | 101 |
| 56 | 100 | 99 |
| 84 | 98 | 97 |
| 112 | 98 | 96 * |

| | | |
|---|---|---|
| (T Test combinaison : * p = 0,05) | | |

Conclusion : les résultats ont montré une diminution moyenne plus importante de la fluorescence de la peau sur la moitié de visage sur laquelle a été appliquée la crème contenant la combinaison selon l'invention (3% (p/p)) que la seconde moitié de visage sur laquelle la crème placebo sans combinaison a été appliquée, avec une efficacité significative pour la combinaison au bout de 4 mois.

### Exemple 5 : Mise en évidence in vivo de l'augmentation de l'éclat du teint de la peau en présence de la combinaison selon l'invention.

Méthode : La mesure in vivo a été effectuée telle que détaillée dans l'exemple 4.

L'augmentation de l'éclat du teint se traduit par une diminution du jaunissement de la peau liée à une diminution de l'accumulation des AGEs. La diminution moyenne de la fluorescence de la peau a été mesurée par chromamétrie (paramètre b) (n = 27).

### Résultats :

| Temps (jours) | Contrôle (%) | Combinaison (%) |
|---|---|---|
| 0 | 100 | 100 |
| 28 | 97,5 | 97 |
| 56 | 97,8 | 96,5 ** |
| 84 | 97,4 * | 94,7 *** |
| 112 | 96,5 ** | 94,2 *** |

| | | |
|---|---|---|
| (T Test combinaison : ** pvalue <0,01 ; *** p<0.001 ; T Test contrôle : * pvalue<0,1, **p<0.05) | | |

### Conclusion :

Les résultats ont montré une diminution moyenne plus importante du jaunissement de la peau, sur la moitié de visage sur laquelle a été appliquée la crème contenant la combinaison selon l'invention, que la crème placebo sans combinaison. La combinaison selon l'invention permet donc d'augmenter la luminosité de la peau du visage.

### Exemple 6 : Exemple d'ingrédient actif cosmétique et/ou dermatologique contenant une combinaison d'un extrait de Salvia miltiorrhiza et de niacine.

Les quantités sont exprimées en pourcentage en poids par rapport au poids total de l'ingrédient actif (% p/p). L'extrait de Salvia miltiorrhiza et la niacine sont préférentiellement obtenus selon l'exemple 1a).

| | |
|---|---|
| Eau | Qsp 100 |
| Extrait de Salvia miltiorrhiza | 0,25-5 |
| Niacine | 1-5 |
| Hexylène glycol | 0,5-5 |
| Caprylyl glycol | 0,1-1 |
| Gomme de xanthane | 0,1-1 |

### Exemple 7: Exemples de compositions cosmétiques et/ou dermatologiques contenant l'ingrédient actif selon l'invention.

*L'ingrédient cosmétique est préparé selon l'exemple 6 précédent. Les quantités indiquées sont en pourcentage en poids par rapport au poids total de la composition.
** L'ingrédient est préparé selon l'exemple 6 précédent. L'ingrédient selon l'invention est stérilisé puis séché avant d'être incorporé dans la composition sous forme de pommade.

### Formulation 7a :

La composition ci-après est préparée selon des méthodes connues de l'Homme du métier, en particulier s'agissant des différentes phases à mélanger entre elles.

| | |
|---|---|
| Ingrédient cosmétique* | 3-5 |
| EDTA | 0,05 |
| Steareth-2 | 2,00 |
| Steareth-21 | 2,50 |
| Alcool cetearyl | 1,00 |
| Caprylate de propylheptyl | 15,00 |
| Hydroxyde de sodium (30 % en solution) | 0,10 |
| Mélange de phénoxyéthanol, chlorphenesin, acide benzoïque, butylène glycol, acide sorbique (Germazide ™ PBS) | 1,25 |
| Mélange de polyacrylate-X, d'd'isohexadécane et de polysorbate | 60 |
| (Sepigel™ SMS 60) | 4,00 |
| Eau | Qsp 100 |

### Formulation 7b : Utilisation de l'ingrédient cosmétique selon l'invention dans une formulation de type eau dans huile

| | | |
|---|---|---|
| A | PEG30 | 3 |
| | Dipolyhydroxystéarate | |
| | Caprique Triglycérides | 3 |
| | Cetearyl Octanoate | 4 |
| | Dibutyle Adipate | 3 |
| | Huile de pépins de raisin | 1,5 |
| | Huile de jojoba | 1,5 |
| | Phenoxyéthanol, Méthylparabène, Propylparabène, Butylparabène, Ethylparabène | 0,5 |
| B | Glycérine | 3 |
| | Butylène Glycol | 3 |
| | Sulfate de magnésium | 0,5 |
| | EDTA | 0,05 |
| | Eau | qsp 100 |
| C | Cyclométhicone | 1 |
| | Diméthicone | 1 |
| D | Parfum | 0,3 |
| E | Ingrédient cosmétique* | 0,001 - 10 % |

### Formulation 7c : Utilisation des produits de l'invention dans une formulation de gels aqueux (Visage)

| | | |
|---|---|---|
| A | Eau | qsp 100 |
| | Carbomère | 0,5 |
| | Butylène Glycol | 15 |
| | Phenoxyéthanol, Méthylparabène, Propylparabène, Butylparabène, Ethylparabène | 0,5 |
| B | Ingrédient cosmétique* | 0,001 - 10 % |

### Formulation 7d : Utilisation des produits de l'invention dans une formulation de type shampoing ou gel douche (Corps)

| | | |
|---|---|---|
| A | Eau | qsp 100 |
| B | Butylène Glycol, Méthylparabène, Ethylparabène, Propylparabène | 0,5 |
| | Phénoxyéthanol, Méthylparabène, Propylparabène, Butylparabène, Ethylparabène | 0,5 |
| C | Acide citrique | 0,8 |
| D | Sodium Laureth Sulfate | 40,0 |
| E | Ingrédient cosmétique* | 0,001 - 10 % |

### Formulation 7e : composition dermatologique sous forme de pommade contenant la combinaison selon l'invention.

La composition ci-après est préparée selon des méthodes connues de l'Homme du métier, en particulier s'agissant des différentes phases à mélanger entre elles.

Les quantités indiquées sont en pourcentage en poids par rapport au poids total de la composition.

| | |
|---|---|
| Ingrédient ** | 3-5 |
| Excipient : | |
| Polyéthylène basse densité | 5,50 |
| Paraffine liquide | Qsp 100 |

### Formulation 7f :

### Glycéryl stéarate, Cétéareth-20,

| | |
|---|---|
| Cétéareth-12, Cétéaryl Alcool, Cétyl Palmitate | 5,00 |
| Béhényl alcool | 2,00 |
| Ethylhexyl palmitate | 5,00 |
| Hexyldécanol, hexyldécyl laurate | 3,00 |
| Dicaprylyl éther | 3,00 |
| Sodium polyacrylate | 0,80 |
| Diméthicone | 1,00 |
| Propylène glycol, phénoxyéthanol, | |
| Chlorphenesine, méthylparabène | 2,50 |
| Glycérine | 3,00 |
| Sodium stéaroyl glutamate | 0,40 |
| Gomme de xanthane | 0,20 |
| Ingrédient actif* | 3,00 |
| Eau | Qsp 100 |

### Formulation 7g :

| | |
|---|---|
| Hexyl laurate | 5,00 |
| Glycérile stéarate, PEG-100 Stéarate | 1,50 |
| Cétéaryl glucoside, cétéaryl alcool | 1,00 |
| Phénoxyéthanol, parabènes | 1,00 |
| Glycérine | 2,50 |
| Carbomère | 1,00 |
| Hydroxyde de sodium | 0,35 |
| Polyacrylamide, C13-C14 isoparaffine, laureth-7 | 0,20 |
| Ingrédient actif* | 5,00 |
| Eau | Qsp 100 |

### Exemple 8 : Mise en évidence in vivo de l'amélioration des propriétés biomécaniques de la peau en présence de la combinaison selon l'invention.

Méthode : La mesure in vivo des propriétés viscoélastiques de la peau a été effectuée à l'aide d'un cutomètre sur la peau du visage d'un échantillon de 39 femmes caucasiennes âgées de 45 ans à 60 ans, ladite peau étant saine, c'est-à-dire constituée de cellules « normales », non pathologiques, ne nécessitant pas de traitement thérapeutique. La mesure a été effectuée comme suit : la formulation 7g (Exemple 7) contenant 5 % en poids de l'ingrédient actif selon l'exemple 6 par rapport au poids total de la formulation (p/p), l'ingrédient actif contenant 0,25 % en poids de l'extrait de feuilles de Salvia miltiorrhiza et 1,2 % en poids de niacine, par rapport au poids total de l'ingrédient actif, a été appliquée sur une moitié de visage 2 fois par jour tous les jours durant 1 mois. Une seconde crème placebo (Contrôle) de même composition mais sans l'ingrédient actif a été appliquée dans les mêmes conditions sur la seconde moitié de visage. Les résultats sont exprimés en pourcentage (%) de variation de la densité optique traduisant la profondeur de pénétration de la peau du cutomètre, donc la fermeté de la peau, au temps t 28 jours par rapport au temps 0. Résultats :

| Temps (jours) | Contrôle (%) | Ingrédient actif (%) |
|---|---|---|
| 0 | 100 | 100 |
| 28 | 97 | 106* |

| | | |
|---|---|---|
| (T Test combinaison : * p <0.05) | | |

Conclusion : l'ingrédient actif contenant la combinaison de Salvia miltiorrhiza et de niacine a augmenté la fermeté de la peau en comparaison du contrôle, au bout de 28 jours d'application bi-quotidienne.

## Revendications

1. Combinaison d'un extrait des parties aériennes de Salvia miltiorrhiza et de niacine et/ou de niacinamide.

2. Combinaison selon la revendication 1, **caractérisée en ce que** l'extrait de Salvia miltiorrhiza est un extrait de feuilles.

3. Combinaison selon l'une quelconque des revendications 1 ou 2, **caractérisée en ce que** l'extrait de Salvia miltiorrhiza est obtenu par extraction aqueuse.

4. Combinaison selon l'une quelconque des revendications 1 à 3, **caractérisée en ce que** l'extrait de Salvia miltiorrhiza est obtenu par extraction dans un solvant, à une concentration comprise entre 1 à 10 %, préférentiellement entre 2% et 3%, en poids de matière fraîche d'au moins une partie de la plante par rapport au poids total de la partie de la plante et du solvant.

5. Combinaison selon l'une quelconque des revendications 1 à 4, **caractérisée en ce que** la niacine et/ou le niacinamide est présente dans la combinaison à une concentration finale entre 1.10⁻⁷ % et 10 %, préférentiellement entre 1.10⁻¹% et 3%, en poids par rapport au poids total de la combinaison.

6. Combinaison selon l'une quelconque des revendications 1 à 5, **caractérisée en ce qu'**elle est dissoute dans une solution aqueuse contenant en outre de l'hexylène glycol, préférentiellement en une teneur de 0,5%, du caprylyl glycol, préférentiellement en une teneur de 1%, et de la gomme de xanthane, préférentiellement en une teneur de 0,5%, en poids par rapport au poids total de la solution aqueuse.

7. Composition cosmétique et/ou nutraceutique comprenant la combinaison selon l'une quelconque des revendications 1 à 6 et au moins un excipient cosmétiquement et/ou nutraceutiquement acceptable.

8. Composition selon la revendication 7, **caractérisée en ce que** l'extrait de Salvia miltiorrhiza est présent dans la composition à une concentration comprise entre 1.10⁻⁴ % et 10 % en poids par rapport au poids total de la composition.

9. Composition selon l'une quelconque des revendications 7 ou 8, **caractérisée en ce que** la niacine et/ou le niacinamide est présente dans la composition à une concentration comprise entre 1.10⁻⁷ % et 10 % en poids par rapport au poids total de la composition.

10. Utilisation non thérapeutique de la combinaison selon l'une quelconque des revendications 1 à 6 ou d'une composition cosmétique et/ou nutraceutique selon l'une quelconque des revendications 7 à 9 pour augmenter la déglycation du collagène et/ou augmenter l'expression du collagène pour maintenir et/ou augmenter la fermeté et/ou l'élasticité de la peau et/ou des muqueuses et/ou du cuir chevelu, et/ou pour prévenir et/ou lutter contre le vieillissement cutané et/ou pour maintenir et/ou augmenter l'éclat du teint de la peau.

11. Utilisation non thérapeutique selon la revendication 10, **caractérisée en ce que** le collagène est le collagène de type I.

12. Utilisation non thérapeutique selon l'une quelconque des revendications 10 ou 11, **caractérisée en ce que** la combinaison ou la composition est appliquée par voie topique sur des parties spécifiques du corps choisies parmi le cou, le décolleté, le ventre, les bras, les cuisses, les hanches, les fesses, la taille et/ou le visage, en particulier le contour des yeux et/ou des lèvres, le front et/ou les zones temporales du cuir chevelu.

13. Procédé de soin cosmétique **caractérisé en ce qu'**il comprend l'application, préférentiellement par voie topique, de la combinaison selon l'une quelconque des revendications 1 à 6 ou de la composition selon l'une quelconque des revendications 7 à 9 sur une zone de peau et/ou de muqueuse et/ou de cuir chevelu.

14. Procédé de soin cosmétique selon la revendication 13, **caractérisé en ce que** ledit procédé est destiné à augmenter la déglycation du collagène et/ou augmenter l'expression du collagène afin de maintenir et/ou augmenter la fermeté et/ou l'élasticité de la peau et/ou des muqueuses et/ou du cuir chevelu et/ou de prévenir et/ou lutter contre le vieillissement cutané et/ou de maintenir et/ou augmenter l'éclat du teint de la peau.

15. Procédé de soin cosmétique selon l'une quelconque des revendications 13 ou 14, **caractérisée en ce que** la zone de peau et/ou de muqueuse et/ou de cuir chevelu est choisie au moins parmi le cou, le décolleté, le ventre, les bras, les cuisses, les hanches, les fesses, la taille et/ou le visage, en particulier le contour des yeux et/ou des lèvres, le front et/ou les zones temporales du cuir chevelu.

16. Combinaison selon l'une quelconque des revendications 1 à 6 ou composition dermatologique la comprenant, éventuellement en association avec un excipient dermatologiquement acceptable, pour son utilisation pour augmenter la déglycation du collagène et/ou augmenter l'expression du collagène, préférentiellement le collagène de type I, pour prévenir et/ou lutter contre les pathologies impliquant une perte de fermeté et/ou d'élasticité de la peau et/ou des muqueuses et/ou du cuir chevelu telles que la couperose, les télangiectasies, l'élastose solaire, la maladie cutix laxa et/ou les vergetures.

17. Composition pharmaceutique, préférentiellement dermatologique, comprenant la combinaison selon l'une quelconque des revendications 1 à 6 éventuellement en association avec un excipient pharmaceutiquement acceptable, préférentiellement applicable par voie topique, pour son utilisation pour augmenter la cicatrisation de la peau et/ou des muqueuses et/ou du cuir chevelu.

## Patentansprüche

1. Kombination von einem Extrakt von oberirdischen Teilen von Salvia miltiorrhiza und von Niazin und/oder von Niazinamid.

2. Kombination nach Anspruch 1, **dadurch gekennzeichnet, dass** der Extrakt von Salvia miltiorrhiza ein Blattextrakt ist.

3. Kombination nach einem der Ansprüche 1 oder 2, **dadurch gekennzeichnet, dass** der Extrakt von Salvia miltiorrhiza durch wässrige Extraktion erhalten wird.

4. Kombination nach einem der Ansprüche 1 bis 3, **dadurch gekennzeichnet, dass** der Extrakt von Salvia miltiorrhiza durch Extraktion in einem Lösungsmittel in einer Konzentration zwischen 1 und 10 Gew.-%, vorzugsweise zwischen 2 Gew.-% und 3 Gew.-% Frischsubstanz mindestens eines Teils der Pflanze, bezogen auf das Gesamtgewicht des Teils der Pflanze und des Lösungsmittels, erhalten wird.

5. Kombination nach einem der Ansprüche 1 bis 4, **dadurch gekennzeichnet, dass** das Niazin und/oder das Niazinamid in der Kombination in einer Endkonzentration zwischen 1 x 10⁻⁷ Gew.-% und 10 Gew.-%, vorzugsweise zwischen 1 x 10⁻¹ Gew.-% und 3 Gew.-%, bezogen auf das Gesamtgewicht der Kombination, vorliegt.

6. Kombination nach einem der Ansprüche 1 bis 5, **dadurch gekennzeichnet, dass** sie in einer wässrigen Lösung gelöst wird, die ferner Hexylenglykol, vorzugsweise in einem Gehalt von 0,5 Gew.-%, Caprylylglykol, vorzugsweise in einem Gehalt von 1 Gew.-%, und Xanthangummi, vorzugsweise in einem Gehalt von 0,5 Gew.-%, bezogen auf das Gesamtgewicht der wässrigen Lösung, enthält.

7. Kosmetische und/oder nutrazeutische Zusammensetzung, umfassend die Kombination nach einem der Ansprüche 1 bis 6 und mindestens einen kosmetisch und/oder nutrazeutisch akzeptablen Hilfsstoff.

8. Zusammensetzung nach Anspruch 7, **dadurch gekennzeichnet, dass** der Extrakt von Salvia miltiorrhiza in der Zusammensetzung in einer Konzentration zwischen 1 x 10⁻⁴ Gew.-% und 10 Gew.-%, bezogen auf das Gesamtgewicht der Zusammensetzung, vorliegt.

9. Zusammensetzung nach einem der Ansprüche 7 oder 8, **dadurch gekennzeichnet, dass** das Niazin und/oder das Niazinamid in der Zusammensetzung in einer Konzentration zwischen 1 x 10⁻⁷ Gew.-% und 10 Gew.-%, bezogen auf das Gesamtgewicht der Zusammensetzung, vorliegt.

10. Nichttherapeutische Verwendung der Kombination nach einem der Ansprüche 1 bis 6 oder einer kosmetischen und/oder nutrazeutischen Zusammensetzung nach einem der Ansprüche 7 bis 9 zum Verstärken der Deglykation des Kollagens und/oder Verstärken der Expression des Kollagens zum Erhalten und/oder Verstärken der Festigkeit und/oder der Elastizität der Haut und/oder von Schleimhäuten und/oder der Kopfhaut und/oder zum Verhindern und/oder Bekämpfen der Hautalterung und/oder zum Erhalten und/oder Verstärken des Glanzes des Hautteints.

11. Nichttherapeutische Verwendung nach Anspruch 10, **dadurch gekennzeichnet, dass** das Kollagen das Typ-I-Kollagen ist.

12. Nichttherapeutische Verwendung nach einem der Ansprüche 10 oder 11, **dadurch gekennzeichnet, dass** die Kombination oder die Zusammensetzung topisch auf spezifische Körperteile aufgebracht wird, die aus dem Hals, dem Dekolleté, dem Bauch, den Armen, den Schenkeln, den Hüften, dem Gesäß, der Taille und/oder dem Gesicht, insbesondere der Augen- und/oder Lippenkontur, der Stirn und/oder den Schläfenbereichen der Kopfhaut, ausgewählt sind.

13. Kosmetisches Pflegeverfahren, **dadurch gekennzeichnet, dass** es das Auftragen, vorzugsweise topisch, der Kombination nach einem der Ansprüche 1 bis 6 oder der Zusammensetzung nach einem der Ansprüche 7 bis 9 auf einen Haut- und/oder Schleimhaut- und/oder Kopfhautbereich umfasst.

14. Kosmetisches Pflegeverfahren nach Anspruch 13, **dadurch gekennzeichnet, dass** das Verfahren zum Verstärken der Deglykation des Kollagens und/oder Verstärken der Expression des Kollagens, um die Festigkeit und/oder die Elastizität der Haut und/oder von Schleimhäuten und/oder der Kopfhaut zu erhalten und/oder zu verstärken und/oder die Hautalterung zu verhindern und/oder zu bekämpfen und/oder den Glanz des Hautteints zu erhalten und/oder zu verstärken, vorgesehen ist.

15. Kosmetisches Pflegeverfahren nach einem der Ansprüche 13 oder 14, **dadurch gekennzeichnet, dass** der Haut- und/oder Schleimhaut- und/oder Kopfhautbereich mindestens aus dem Hals, dem Dekolleté, dem Bauch, den Armen, den Schenkeln, den Hüften, dem Gesäß, der Taille und/oder dem Gesicht, insbesondere der Augen- und/oder Lippenkontur, der Stirn und/oder den Schläfenbereichen der Kopfhaut, ausgewählt ist.

16. Kombination nach einem der Ansprüche 1 bis 6 oder dermatologische Zusammensetzung, die diese umfasst, eventuell in Verbindung mit einem dermatologisch akzeptablen Hilfsstoff, zu deren Verwendung zum Verstärken der Deglykation des Kollagens und/oder Verstärken der Expression des Kollagens, vorzugsweise des Typ-I-Kollagens, zum Verhindern und/oder Bekämpfen von Pathologien, die einen Verlust der Festigkeit und/oder der Elastizität der Haut und/oder von Schleimhäuten und/oder der Kopfhaut mit sich bringen, wie Couperose, Teleangiektasien, solare Elastose, Cutis-laxa-Syndrom und/oder Dehnungsstreifen.

17. Pharmazeutische, vorzugsweise dermatologische Zusammensetzung, umfassend die Kombination nach einem der Ansprüche 1 bis 6, eventuell in Verbindung mit einem pharmazeutisch akzeptablen Hilfsstoff, vorzugsweise topisch auftragbar, zu deren Verwendung zum Verstärken der Heilung der Haut und/oder von Schleimhäuten und/oder der Kopfhaut.

## Claims

1. Combination of an extract of the aerial parts of Salvia miltiorrhiza and niacin and / or niacinamide.

2. Combination according to claim 1, **characterized in that** the Salvia miltiorrhiza extract is an extract of leaves.

3. Combination according to any one of claims 1 or 2, **characterized in that** the Salvia miltiorrhiza extract is obtained by aqueous extraction.

4. Combination according to any one of claims 1 to 3, **characterized in that** the Salvia miltiorrhiza extract is obtained by extraction in a solvent, at a concentration of between 1% and 10%, preferentially of between 2% and 3%, by weight of fresh material of at least part of the plant in relation to the total weight of the part of the plant and the solvent.

5. Combination according to any one of claims 1 à 4, **characterized in that** the niacin and / or niacinamide is present in the combination at a final concentration between 1x10⁻⁷% and 10%, preferentially between 1 × 10⁻¹% and 3%, by weight relative to the total weight of the combination.

6. Combination according to any one of claims 1 to 5, **characterized in that** it is dissolved in an aqueous solution further containing hexylene glycol, preferentially at a content of 0.5%, caprylyl glycol, preferentially at a content of 1%, and xanthan gum, preferentially at a content of 0.5%, by weight relative to the total weight of the aqueous solution.

7. Cosmetic and / or nutraceutical composition comprising the combination according to any one of claims 1 to 6 and at least one cosmetically and / or nutraceutically acceptable excipient.

8. Composition according to claim 7, **characterized in that** the Salvia miltiorrhiza extract is present in the composition at a concentration of between 1x10⁻⁴% and 10% by weight relative to the total weight of the composition.

9. Composition according to any one of claims 7 or 8, **characterized in that** the niacin and / or niacinamide is present in the composition at a concentration of between 1x10⁻⁷% and 10% by weight relative to the total weight of the composition.

10. Non therapeutic use of the combination according to any one of claims 1 to 6 or a cosmetic and / or nutraceutical composition according to any one of claims 7 to 9 to increase the deglycation of collagen and / or increase the expression of collagen to maintain and / or increase the firmness and / or elasticity of the skin and / or mucous membranes and / or scalp, and / or to prevent and / or fight against skin aging and / or to maintain and / or increase the radiance of the complexion of the skin.

11. Non therapeutic use according to claim 10, **characterized in that** the collagen is type I collagen.

12. Non therapeutic use according to any one of claims 10 or 11, **characterized in that** the combination or composition is applied topically to specific parts of the body selected from the neck, neckline, belly, arms, thighs, hips, buttocks, waist and / or face, in particular the contour of the eyes and / or the lips, the forehead and / or the temporal region of the scalp.

13. Cosmetic treatment process **characterized in that** it comprises the application, preferably topically, of the combination according to any one of claims 1 to 6 or the composition according to any one of claims 7 to 9 on an area of the skin and / or mucous membrane and / or scalp.

14. Cosmetic treatment method according to claim 13, **characterized in that** said method is intended to increase the deglycation of collagen and / or to increase the expression of collagen in order to maintain and / or increase the firmness and / or elasticity of the skin and / or mucous membranes and / or scalp and / or to prevent and / or fight against skin aging and / or to maintain and / or increase the radiance of the complexion of the skin.

15. Cosmetic treatment method according to any one of claims 13 or 14, **characterized in that** the area of the skin and / or mucous membrane and / or scalp is selected at least from the neck, neckline, belly, arms, thighs, hips, buttocks, waist and / or face, in particular the contour of the eyes and / or the lips, the forehead and / or the temporal region of the scalp.

16. Combination according to any one of claims 1 to 6 or a dermatological composition comprising it, optionally in combination with a dermatologically acceptable excipient, for its use for increasing the deglycation of collagen and / or increase the expression of collagen, preferentially of type I collagen, to prevent and / or fight against pathologies involving loss of firmness and / or elasticity of the skin and / or mucous membranes and / or scalp such as rosacea, telangiectasia, solar elastosis, cutix laxa disease and / or stretch marks.

17. Pharmaceutical composition, preferentially dermatological, comprising the combination according to any one of claims 1 to 6 optionally in combination with a pharmaceutically acceptable excipient, preferentially topically applicable, for its use for increasing the healing of the skin and / or mucous membranes and / or scalp.
